**Europäisches Patentamt**

**European Patent Office**

**Office européen des brevets**

(19)

(11) **EP 1 249 704 A1**

(12) **EUROPÄISCHE PATENTANMELDUNG**

(43) Veröffentlichungstag:
**16.10.2002 Patentblatt 2002/42**

(51) Int Cl.$^7$: **G01N 33/68**

(21) Anmeldenummer: **01109232.7**

(22) Anmeldetag: **14.04.2001**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(71) Anmelder:
- **Frank, Hans-Georg c/o Lehrstuhl für Anatomie II Universitätsklinikum der RWTH 52057 Aachen (DE)**
- **Kaufmann, Peter c/o Lehrstuhl für Anatomie II Universitätsklinikum der RWTH 52057 Aachen (DE)**

(72) Erfinder:
- **KAUFMANN Peter 6291 CX Vaals (NL)**
- **FRANK, Hans-Georg 6462 EL Kerkrade (NL)**

- **SCHMITZ Ulrike 52070 Aachen (DE)**

(74) Vertreter:
**Werner, Hans-Karsten, Dr.Dipl.-Chem. et al Patentanwälte Von Kreisler-Selting-Werner Postfach 10 22 41 50462 Köln (DE)**

Bemerkungen:
Das Sequenzprotokoll, das als Anlage zu den Anmeldungsunterlagen mitveröffentlicht ist, ist nach dem Anmeldetag eingereicht worden. Der Anmelder hat erklärt, dass dieses nicht über den Inhalt der Anmeldung in der ursprünglich eingereichten Fassung hinausgeht.

(54) **Verfahren zur Selektion von immunologischen Bindungsmolekülen**

(57) Verfahren zur Selektion von immunologischen Bindungsmolekülen aus einer Bibliothek immunologischer Bindungsmoleküle mit folgenden Schritten:

- In Kontakt bringen der Bibliothek immunologischer Bindungsmoleküle mit einer zweidimensional angeordneten Probe enthaltend mindestens ein Epitop

- Isolierung der an das mindestens eine Epitop gebundenen immunologischen Bindungsmoleküle.

Fig. 1

SEQUENCE LISTING

<110> Frank, Hans-Georg

<120> Verfahren zur Selektion von immunologischen
Bindungsmolekülen

<130> 003166ep

<140> EP01109232.7
<141> 2001-04-14

<160> 6

<170> PatentIn Ver. 2.1

<210> 1
<211> 38
<212> DNA
<213> Artificial Sequence

<220>
<223> Description of Artificial Sequence: Primer CSCVK

<400> 1
gtggcccagg cggccctgac tcagccgtcc tcggtgtc                    38

<210> 2
<211> 34
<212> DNA
<213> Artificial Sequence

<220>
<223> Description of Artificial Sequence: Primer CKJo-B

<400> 2
cgaagatcta gaggactgac ctaggacggt cagg                    34

<210> 3
<211> 39
<212> DNA
<213> Artificial Sequence

<220>
<223> Description of Artificial Sequence: Primer CSVHo-F

```
<400> 3
ggtcagtcct ctagatcttc cgccgtgacg ttggacgag                    39


<210> 4
<211> 44
<212> DNA
<213> Artificial Sequence

<220>
<223> Description of Artificial Sequence: Primer CSCG-B

<400> 4
ctggccggcc tggccactag tggaggagac gatgacttcg gtcc             44


<210> 5
<211> 42
<212> DNA
<213> Artificial Sequence

<220>
<223> Description of Artificial Sequence: Primer CSC-F

<400> 5
gaggaggagg aggaggaggt ggcccaggcg gccgtgactc ag               42


<210> 6
<211> 47
<212> DNA
<213> Artificial Sequence

<220>
<223> Description of Artificial Sequence: Primer CSC-B

<400> 6
gaggaggagg aggaggagga gctggccggc ctggccacta gtggagg          47
```

**Beschreibung**

Die vorliegende Erfindung betrifft ein Verfahren zur Selektion von immunologischen Bindungsmolekülen.

Es ist in der Biochemie üblich zur Charakterisierung einer unbekannten, in einem Organismus vorkommenden Substanz, beispielsweise eines Proteins, geringe Menge der Substanz möglichst weitgehend aufzureinigen, um dann anschließend gegen die Substanz Antikörper zu erzeugen. Die so erhaltenen poly- oder monoklonalen Antikörper können dann zur weiteren Aufreinigung der Substanz eingesetzt werden. Der erhaltene Antikörper kann insbesondere auch dazu benutzt werden, die Größe der Substanz in Blots von gelelektrophoretischen Trennungen oder die Verteilung im Organismus an histologischen Schnitten zu untersuchen.

Hier tritt jedoch das Problem auf, dass die Substanzen, beispielsweise während einer elektrophoretischen Trennung oder bei der Anfertigung eines histologischen Schnittes, in ihrer Struktur so verändert werden, dass Antikörper, die gegen die aufgereinigten, strukturell nicht veränderten Substanzen gewonnen werden, die teilweise oder vollständig denaturierten Substanzen in Blots oder Schnitten nicht mehr erkennen.

Auf der anderen Seite gibt es viele Organe und Gewebe mit zahlreichen Subkompartimenten und Zelltypen, für die spezifische Proteine und zugehörige Antikörper bisher nicht beschrieben sind. Darüber hinaus ist die genaue gewebe- bzw. zellartspezifische Verteilung vieler Substanzen nicht bekannt und muss erst ermittelt werden.

Aufgabe der vorliegenden Erfindung ist daher, eine Verfahren bereitzustellen, mit dem in einfacher Weise immunologische Bindungsmoleküle insbesondere Antikörper gegen Substanzen erhalten werden können, ohne das die erwähnten Nachteile auftreten. Gelöst wird die Aufgabe durch ein Verfahren mit den Merkmalen des Patentanspruches 1.

Das erfindungsgemäße Verfahren zur Selektion von immunologischen Bindungsmolekülen aus einer Bibliothek immunologische Bindungsmoleküle umfasst folgende Schritte:

- In Kontakt bringen der Bibliothek immunologischer Bindungsmoleküle mit einer zweidimensional angeordneten Probe enthaltend mindestens ein Epitop

- Isolierung der an das mindestens eine Epitop gebundenen immunologischen Bindungsmoleküle.

"Immunologische Bindungsmoleküle" sind Moleküle, die direkt oder indirekt durch Immunisierung erhalten werden und andere Moleküle binden können. Der Begriff umfasst insbesondere Antikörper und Antikörperfragmente, sowie single chain Antikörper.

"Bibliothek immunologischer Bindungsmoleküle" bedeutet eine Vielzahl von verschiedenen immunologischen Bindungsmolekülen, die noch so mit ihrer DNA Informationen verbunden sind, dass durch Vereinzelung von Mitgliedern monoklonale immunologische Bindungsmoleküle erhalten werden können. Der Begriff umfasst insbesondere Bibliotheken von single chain Antikörpern in Phagen-Bibliotheken.

"Epitop" ist eine Struktur, die von einem immunologischen Bindungsmolekül erkannt werden kann.

"Selektion immunologischer Bindungsmoleküle" bedeutet ein Verfahren, bei dem die Bindungsfähigkeit der Bindungsmoleküle an Substanzen getestet wird und die Moleküle isoliert werden, die besonders hohe Bindungsaffinitäten zeigen.

Das erfindungsgemäße Verfahren geht von einer bestehenden Bibliothek immunologischer Bindungsmoleküle aus. Solche Bibliotheken können durch Verfahren erhalten werden, wie sie zum Beispiel in Barbas CF 3$^{rd}$, Furton DR, Scott JK, Silverman GJ (2001) Phage Display: A laboratory manual, 1$^{st}$ edn. CSHL Press, Cold Spring Harbor, New York beschrieben sind.

Es ist besonders vorteilhaft, wenn solche Bibliotheken aus Nicht-Säugetieren gewonnen werden, da diese gegen hochkonservierte Epitope von Säugetieren bessere Antikörper bilden, als beispielsweise Mäuse, die üblicherweise für Immunisierung verwendet werden. Bevorzugte Nicht-Säugetiere sind Vögel, insbesondere Hühner.

Als zweidimensional angeordnete Proben eignen sich insbesondere histologische Schnitte oder Blots von Naturstoffelektrophoresen.

Zur Erstellung von geeigneter histologischen Proben eignen sich alle klassischen histologischen Verfahren der Einbettung, insbesondere durch Einfrieren oder durch Fixieren mit Paraffin- oder Kunststoffeinbettung, gefolgt vom Anfertigen dünner (0,5 bis 10 μm) Gewebsschnitte. Solche Verfahren sind z.B. beschrieben in Romeis B (1989) Mikroskopische Technik, 17. Aufl., München. Dabei werden die Proteine im Schnitt in genau der Verteilung wiedergefunden, die sie auch im lebenden Organismus bei der Erfüllung ihrer Funktion hatten. Durch die Fixierung können sie jedoch in ihrer Struktur verändert werden.

Es kann sich bei der zweidimensional angeordneten Probe um den Blot einer Naturstoffelektrophorese handeln. Um eine möglichst hohe Auflösung der Substanzen zu erzielen, wird die Elektrophorese möglichst zweidimensional durchgeführt. Geeignete Stoffe für solche Elektrophoresen sind u.a. Nukleinsäuren und Proteine. Die zweidimensional aufgetrennten Proteine, beispielsweise nach Molekulargewicht und isoelektrischen Punkten werden durch übliche Verfahren auf eine sog. Blotmembran übertragen. Verfahren hierzu sind beispielsweise beschrieben in Harlow E., Lane D (1988) Antibodies: A laboratory manual. 1st edn. CSHL Press, Cold Spring Harbor, New York.

Im Falle solcher zweidimensionalen Gele ist es besonders bevorzugt, Gele aus verschiedenen biologischen Kontexten zu vergleichen, beispielsweise nichtinvasive gegen invasive Zellen, tumorigene gegen nicht-tumorigene Zellen, Stammzellen gegen differenzierte Zellen, primär isolierte Zellen gegen Zelllinien etc. Hierbei lässt sich aus den Blots zunächst nur entnehmen, dass unterschiedliche Substanzen vorhanden sind; die Struktur der Naturstoffe, beispielsweise Proteine, ist in der Regel nicht bekannt.

Zur Selektion der Bibliothek immunologischer Bindungsmoleküle wird nun die zweidimensional angeordnete Probe, beispielsweise der histologische Schnitt oder die Blotmembran mit der Bibliothek so in Kontakt gebracht, dass die immunologischen Bindungsmolekülen Bindungen eingehen können.

In Abhängigkeit von der Probe ist es ggf. vorteilhaft, vorher die zweidimensional angeordnete Probe zu blokkieren, um unspezifische Bindung der Bindungsmoleküle zu verhindern. Solche Blockierungsschritte, beispielsweise mit Albuminlösung sind dem Fachmann bekannt.

Nach der erfolgten Bindung der Bindungsmoleküle werden Waschschritte durchgeführt, die zwischen unspezifisch und spezifisch gebundenen Bindungsmolekülen differenzieren sollen, d.h. die nur schwach unspezifisch gebundenen Moleküle werden entfernt.

Aus der zweidimensional angeordneten Probe werden nun mit geeigneten Mitteln die Schnitt-Areale oder die Zonen in der Blotmembran entfernt gegen die immunologische Bindungsmoleküle gewonnen werden sollen. Hierzu können Mikrodissektionseinrichtungen verwendet werden, wobei sowohl Glaskapillare und mechanische Bearbeitung als auch Lasertechniken zum Einsatz kommen können. Solche Einrichtungen sind beispielsweise kommerziell erhältlich von den Firmen eppendorf (Hamburg) bzw. SL Microtest (Jena).

Die an diese Areale gebundenen immonulogische Bindungsmoleküle werden nun verwendet, um in einer an die Bibliothek angepassten Weise amplifiziert zu werden.

Je nach der bereits im ersten Selektionsschritte gefundenen Spezifität kann es sinnvoll sein, das Selektionsverfahren ein oder mehrmals zu wiederholen.

Als Ergebnis dieses Verfahrens werden immunologische Bindungsmoleküle erhalten. Die Spezifität der Bindungsmoleküle ist durch ihre Fähigkeit definiert, bestimmte Regionen in zweidimensionalen Proben zu binden, d.h. die Moleküle zeigen eine topographisch definierte Primärspezifität.

Es kann auch sinnvoll sein, um immunologische Bindungsmoleküle zu selektieren, die für die zwei Proben unterscheidenden Merkmale spezifisch sind "einen Teil der" immunologischen Bindungsmoleküle durch in Kontakt bringen einer ersten zweidimensional angeordneten Probe mit der Bibliothek immunologischer Bindungsmoleküle zu entfernen, um dann durch in Kontakt bringen mit einer zweiten zweidimensionalen angeordneten Proben immunologische Bindungsmoleküle für solche Epitope zu selektieren, die die erste zweidimensional angeordnete Probe und die zweidimensional angeordnete Probe unterscheiden.

Das erfindungsgemäße Verfahren wird durch die folgenden Beispiele weiter beschrieben:

_Beispiel 1:_

Die folgenden Ausführungen stellen ein Beispiel für die Anwendung des erfindungsgemäßen Verfahrens dar. Das Beispiel umfasst die Schritte der Immunisierung eines Nicht-Säugetieres mit verschiedenen Antigen-Präparationen, die Erstellung einer Phagen-Bibliothek sowie die Selektion spezifischer immunologischer Bindungsmoleküle im Sinne des Verfahrens.

**_Immunisierung der Tiere_**

12 "white leghorn" Hühner im Alter von 6 bis 18 Monaten wurden immunisiert. Die Immunisierungsprotokolle wurden auf publizierten Standardschemata aufgebaut (Gassmann, M. et al. _FASEB J_ (1990) 4:2528-2532) und umfassten eine erste Injektion einer Antigenpräparation (siehe Tabelle 2) in komplettem Freund'schem Adjuvans, die von zwei Booster-injektionen im Abstand von jeweils 2 bis 4 Wochen gefolgt wurden. Booster-Injektion wurden mit inkomplettem Freund' schem Adjuvans durchgeführt. Falls mit lebenden menschlichen Zellen immunisiert wurde, wurden pro Injektion eine Million Zellen ohne Adjuvans verabreicht (siehe auch Tabelle 2). 5 Tage nach der letzten Injektion wurden die immunisierten Tiere getötet, die Milzen entfernt und in steriler isotoner Lösung ins Labor verbracht.

**_Präparation von Splenozyten, Gesamt-RNA und cDNA_**

Unter sterilen Bedingungen wurde die Milz in 8-10 kleine Stücke zerteilt. Diese Stücke wurden in einem Elvehjem Glasröhrchen mit Hilfe des passenden Pistills vorsichtig von Hand weiter in 3 ml steriler isotoner Salzlösung suspendiert. Diese Suspension wurde durch ein Edelstahlsieb (150 mesh) filtriert, die im Filtrat enthaltenen Milzzellen pelletiert (400g, 5 min. Raumtemperatur) und anschließend in Lyse-Puffer (0.15 M $NH_4Cl$; 1 mM $KHCO_3$; 0.1 mM $Na_2EDTA$; pH 7.2) die Erythrozyten selektiv lysiert. Aus den verbleibenden Splenozyten wurde die Gesamt-RNA präpariert sowie cDNA nach einer reversen Transkription mit oligo-dT Primern hergestellt.

### Polymerase-Ketten Reaktionen (PCR) für die Bibliothekserstellung

Primer, die benutzt werden können, um die variablen Regionen von leichter (Vk) und schwerer Kette (Vh) der Immunglobulin-cDNAs zu amplifizieren, sind in Tabelle 1 zusammengefasst (Andris-Widhopf, J., Rader, C., Steinberger, PI, Fuller, R., Barbas, C.F., III (2000) Methods for the generation of chicken monoclonal antibody fragments by phage display. J. Immunol. Methods 242, 159-181). Die PCR wurde mit folgenden Parametern durchgeführt: Die initiale Denaturierung wurde für 1 min bei 94°C durchgeführt, gefolgt von 30 Zyklen mit 15 s Denaturierung (94°C), 15 s Annealing (56°C) und 90 s Elongation (74°C). Die Primer führen einen Überlappungsbereich ein, der für die splice overlap extension PCR bei der Erstellung der für scFv codierenden Abschnitte benötigt wird. Die PCR führt zu Produkten von ca. 350 bp Größe. Nach gründlicher Reinigung der Produkte wurden diese für die splice overlap extension PCR eingesetzt.

### Splice overlap extension PCR

Äquimolare Mengen (je 100 ng) der Amplifikate von Vk und Vh wurden in der splice overlap extension PCR eingesetzt. Weiterhin enthielten die Reaktionsmischungen ein Paar speziell definierter Primer (Siehe Tabelle 1; Andris-Widhopf, J., Rader, C., Steinberger, PI, Fuller, R., Barbas, C.F., III (2000) Methods for the generation of chicken monoclonal antibody fragments by phage display. J. Immunol. Methods 242, 159-181), zusammengefasst, die unter anderem die für die Restriktion und Ligation benötigten Restriktionsschnittstellen in das Produkt einführen. Nach initialer Denaturierung bei 94°C für 1 min. folgten 35 Zyklen mit 15 s Denaturierung (94°C), 15 s Annealing (56°C) und 120 s Elongation (74°C). Das gewünschte Produkt hat eine Größe von ca. 750 bp.

### Vektor und Ligation

Verwendet wurde der Vektor pComb3H-SS (Barbas, CF 3rd. *Curr. Opin. Biotechnol.* (1993) 4:526-530, Biassoni, R. et al. *Semin. Cancer Biol.* (1999) 9:13-18, Siegel, D.L. et al. *J Immunol. Methods* (1997) 206:73-85, Andris-Widhopf, J., Rader, C., Steinberger, PI, Fuller, R., Barbas, C.F., III (2000) Methods for the generation of chicken monoclonal antibody fragments by phage display. J. Immunol. Methods 242, 159-181).
Dieser Vektor sowie die oben beschriebenen Primer wurden im Scripps Research Institut, La Jolla, Kalifornien, USA, speziell für das Phage Display von Antikörperbibliotheken hergestellt und entworfen.
Vor der Ligation wurde der Vektor durch Verdau mit *Sfi* I linearisiert. Da der Vektor zwei asymetrische Schnittstellen dieses Restriktionsenzyms enthält, ist nach Verdau und Reinigung des Vektors eine direktionelle Klonierung direkt ohne weiteren Verdauungsschritt möglich. Parallel dazu wurde auch das Produkt der splice overlap extension PCR mit dem Enzym verdaut und das restringierte Produkt gereinigt.

### Erstellung der Phagen-Antikörper Bibliotheken

Präparierter Vektor (14 µg) und PCR-Produkt (10 µg) wurden zusammen in der Gegenwart von 20 U T4-ligase über Nacht bei 4°C inkubiert, die Reaktionsprodukte mit Ethanol präzipitiert und in 50 µl Aqua Bidestillata resuspendiert. Mit dieser DNA Lösung wurden elektrokompetente E.coli (XL-1 Blue) transformiert. Die Anzahl der Transformanten wurde durch Titerung auf LB-Ampicillin Platten bestimmt (Sambrook,J., E.F.Fritsch, and T.Maniatis. Cold Spring Harbor Laboratory (1989), Cold Spring Harbor, N.Y.) und die Phagen-Bibliotheken nach Superinfektion der transformierten E. coli mit dem Helferphagen M13KO7 geerntet. Die in Phagenform vorliegenden Bibliotheken wurden sowohl einzeln als auch als gemischte Gesamtbibliothek (kumulative Bibliothek) gelagert.

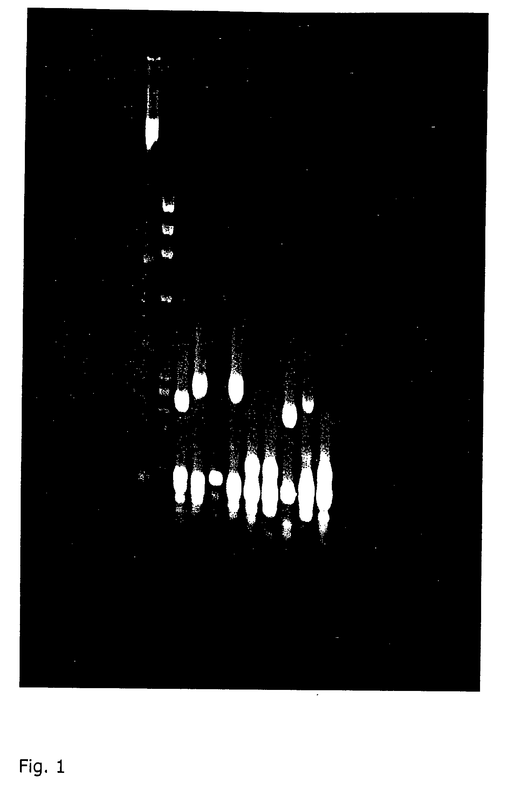

Um einen Eindruck von der Diversität der Bibliothek zu bekommen und die Dominanz einiger weniger Klone auszuschließen, wurden die Bandenmuster von 9 zufällig gezogenen Klonen nach Restriktion des Inserts analysiert (s. Figur 1). Zur Überprüfung der Diversität der kumulativen Bibliothek wurden 9 Klone willkürlich gepickt, die Plasmide isoliert und die Inserts in einer PCR amplifiziert. Die PCR-Produkte wurden gereinigt, mit M*sp*I restringiert, in einem 2% Agarosegel aufgetrennt und mit Ethidiumbromid gefärbt. Die Probenreihen sind von links nach rechts wie folgt in aufsteigender Nummerierung angeordnet. Reihe 1: 100 bp Größen-Leiter; Reihe 2: ΦX174/H*ae*III; Reihe 3-11: Klone 1-9. Dies zeigt, dass die Bibliothek nicht von wenigen Klonen dominiert wird.

*Beispiel 2:*

**Erstellung von zweidimensional angeordneten Proben (2-D PAGE)**

**1.1 Proben Vorbereitung**

Die Zelllysate der Chorionkarzinomzelllinien JEG-3 und AC1-1 sowie die der Hybridzellinien AC-1M32, AC-1M46, AC-1M59, AC-1M81, AC-1M88 (s. Deutsche Sammlung für Mikroorganismen und Zellkulturen, DSMZ, Braunschweig) werden verwendet.

Die Zellen je einer konfluenten großen Flasche (75qcm Kulturfläche) werden mit einem Zellschaber abgelöst und bei 1500 rpm, 5', 4°C pelletiert. Danach werden die Zellen zweimal mit je 10ml PBS gewaschen. Die Zellen werden in 1,5ml PBS aufgenommen, in ein 1,5ml Reaktionsgefäß überführt und bei 3000 rpm, 5', RT pelletiert. Nach der Zugabe von 200µl Lyse-Puffer werden die Zellen mit einem Pellet-Mischer für 1' bei voller Geschwindigkeit homogenisiert. Anschließend werden Zelltrümmer bei 17500xg, 45', 4°C pelletiert. Der Überstand wird in ein neues Reaktionsgefäß überführt und dieses auf Eis gestellt.

**1.2 Proteingehaltsbestimmung**

Die Proteinbestimmung erfolgt nephelometrisch. Die Eichgerade wird mit BSA aufgestellt, d.h. die gemessenen Werte entsprechen Protein in BSA-Äquivalenten.

Die Proben werden für die Proteinbestimmung 1:5 mit Lyse-Puffer verdünnt und dann je 2µl in eine dreifache Bestimmung eingesetzt.

| Ansatz | für 190µl dest. Wasser |
|---|---|
| Proteinbestimmung : | 8µl Lyse-Puffer |
| | 2µl Probe 1:5 verdünnt |
| | vortexen, kurz zentrifugieren |
| | 200µl Trichloressigsäure 20% |
| | hinzufügen, vortexen. |

Die Proben werden eine Stunde auf Eis inkubiert, in Halbmikroküvetten überführt und in einem Behring Laser-Nephelometer gemessen.

Nach der Proteinbestimmung werden die Proben in Portionen zu 150µg Protein bei -70°C eingefroren.

**1.3 Rehydratisierung der ImmobilineDryStrips**

Jeder Gelstreifen wird in insgesamt 360µl Lösung im *Reswelling Tray* (Amersham Pharmacia Biotech) rehydratisiert.

Die Lösung setzt sich aus der Probe in Lyse-Puffer (Urea 7M, Thiourea 2M,CHAPS 4%(w/v), Tris 40mM), dem Marker und der Rehydratisierungslösung (Urea 8M, CHAPS 2%(w/v), Pharmalyte 3-10 0,5%(v/v), DTT 0,2%(w/v), Orange G 0,2mg; Lösung ansetzen in cØmplete ™ Protease Inhibitior Cocktail; vor Gebrauch steril filtrieren) zusammen. Als Marker für die erste Dimension werden 15µl der Carbanhydrase aus dem Carbamylyte Calibration Kit (Amersham Pharmacia Biotech) verwendet. Die Rehydratisierung erfolgt in der Regel über Nacht, mindestens aber zehn Stunden bei 4°C.

**1.4 Isoelektrische Fokussierung**

Die isoelektrische Fokussierung wird in dem IPG *Strip Kit* der Multiphor II-Elektrophorese-Kammer (AmershamPharmacia Biotech) durchgeführt Für die Zelllysate haben sich die *ImmobilineDryStrips* (Amersham Pharmacia Biotech)der Länge 18cm und dem linearen pH-Bereich von 3-10 bzw. 4-7 (für Proteine mit saurem isoelektrischem Punkt (IEP)) als geeignet erwiesen.

Während des Laufes werden die Gelstreifen mit Silikonöl bedeckt.

Die Fokussierung im basischen Bereich wird durch fehlendes Reduktionsmittel beeinträchtigt. Da DTT (Dithiothreitol) bei der Elektrophorese zur Anode wandert, wird vor der Kathode ein DTT-Depot angelegt. Dazu wird ein IEF-Filterpapierstreifen auf 11cm gekürzt und mit 500µl DTT-Lösung getränkt. Überschüssige Flüssigkeit wird entfernt.

Die Fokussierungsbedingungen werden durch das Netzgerät EPS 3500 XL von Amersham Pharmacia Biotech kontrolliert. Das Netzgerät wird auf automatische Durchführung der folgenden Fokussierungsphasen im Gradientenmodus eingestellt:

Tabelle 1:

| Programm des Netzgerätes für die Focussierung im pH- Gradient 3-10 | | | | |
|---|---|---|---|---|
| | Spannung | Stromstärke | Leistung | Dauer |
| Phase 1: | 150V | 1mA | 5W | 1Vh |
| Phase 2: | 150V | 1mA | 5W | 150Vh |
| Phase 3: | 300V | 1mA | 5W | 1Vh |
| Phase 4: | 300V | 1mA | 5W | 300Vh |
| Phase 5. | 3500V | 1mA | 5W | 10000Vh |
| Phase 6: | 3500V | 1mA | 5W | 10000Vh |

Tabelle 2:

| Programm des Netzgerätes für die Focussierung im pH- Gradient 4-7 | | | | |
|---|---|---|---|---|
| | Spannung | Stromstärke | Leistung | Dauer |
| Phase 1: | 150V | 1mA | 5W | 1Vh |
| Phase 2: | 150V | 1mA | 5W | 150h |
| Phase 3: | 300V | 1mA | 5W | 1Vh |
| Phase 4: | 300V | 1mA | 5W | 600Vh |
| Phase 5: | 600V | 1mA | 5W | 1Vh |
| Phase 6: | 600V | 1mA | 5W | 600Vh |
| Phase 7: | 3500V | 1mA | 5W | 3000Vh |
| Phase 8: | 3500V | 1mA | 5W | 30000Vh |

Nach der Fokussierung können die Gelstreifen bei -70°C einige Zeit gelagert werden.

## 1.5 SDS-PAGE

Die zweite Dimension ist eine horizontale SDS-PAGE. Die Gele haben eine Größe von 22x24,5cm und werden auf einem Kunststofffilm gegossen. Das hat den Vorteil, daß sie beim Trocknen nicht schrumpfen. Gegossen werden Gradientengele mit einem Gradienten von 12% bis 14% Acrylamid. Vor dem Gießen der Gele werden die Kunststofffilme sechsmal zehn Minuten mit dest. Wasser gewaschene.
Die Gele für die zweite Dimension waren wie folgt zusammengesetzt:

| | Sammelgel | Trenngel Lsg. | Trenngel Lsg. |
|---|---|---|---|
| | T=6%, C=3% | 1 | 2 |
| | | T=12%, | T=14%, |
| | | C=2% | C=0,5% |
| Acrylamid 40% | 1,82ml | 4,365ml | 5,1ml |
| N,N'-Methylenbisacrylamid 2% | 1,125ml | 2,7ml | 0,525ml |
| Glycerin 87% | 6,5ml | 4,3ml | - |
| Gelpuffer 10x | 1,5ml | 1,5ml | 1,5ml |
| Dest. Wasser | 4,05ml | 2,125ml | 7,875ml |
| TEMED | 10μl | 10μl | 10μl |
| APS 40% | 18μl | 10,6μl | 7,7μl |
| Endvolumen | 15ml | 15ml | 15ml |

Die vor dem Gellauf erforderliche Äquilibrierung erfolgt in zwei Schritten im Äquilibrierungspuffer (Urea (6M), Tris-HCl (50mM pH 8,5), SDS (2%(w/v)), Glycerin (30%(w/v))). Im ersten Schritt werden die Proteine mit Hilfe von 1% (w/v) DTT (Dithiothreitol) im Puffer reduziert. Danach wird das überschüssige Reduktionsmittel durch 4%(w/v) 2-Iodacetamid im Puffer entfernt. Beide Puffer enthalten zusätzlich Bromphenolblau zur Markierung der Lauffront. Jeder Schritt dauert 15' und wird auf einem Schüttler durchgeführt. Vor dem Gebrauch müssen beide Lösungen filtriert werden.

Nach dem zweiten Schritt werden die Gelstreifen eine Sekunde mit dest. Wasser abgespült und dann der überschüssigen Puffer ca. drei Minuten von den Gelstreifens herunter laufen gelassen.

Zum Starten des Gellaufs werden die Gelstreifen mit der Seite pH 3 bzw. pH 4 nach links auf die Gele für die zweite Dimension aufgelegt. Für den Laufpuffer werden die *ExcelGel SDS Buffer Strips* verwendet. Die Pufferstreifen enthalten Tricin als Folge-Ion.

Als Marker für die zweite Dimension wird der *Mark12™ Wide Range Protein Standard* von Novex, San Diego, USA verwendet. Neben den Gelstreifen werden jeweils 4μl unverdünnt aufgetragen.

Skizze :

M    pH3 ⟵ 1D ⟶ pH10   M

2D

Laufbedingungen :

Die Bedingungen des Gellaufs werden durch das Netzgerät EPS 3500 XL von Amersham Pharmacia Biotech kontrolliert. Das Netzgerät wird auf automatische Durchführung der folgenden Phasen eingestellt:

Tabelle 3:

| Programm des Netzgerätes für die SDS-PAGE | | | | |
|---|---|---|---|---|
| | Spannung | Stromstärke | Leistung | Dauer |
| Phase 1: | 100V | 20mA | 50W | 1h20' |
| Phase 2: | 600V | 30mA | 50W | 5h |

Nach der ersten Phase wird der Lauf kurz unterbrochen und die Gelstreifen werden vom Gel entfernt, um den Kathoden-Pufferstreifen auf die Stelle zu verschieben, wo vorher der Gelstreifen lag. Nach dem Ende des Programmes wird das Geld von dem Kunststoffträger gelöst, nach Standardverfahren (Harlow E, Lane D (1988) Antibodies :A laboratory manual. 1st edn. CSHL Press, Cold Spring Harbor, New York) in elektrolythaltige Filterpapierstreifen eingelegt und auf eine Polyvinylidendifluorid (PVDF) Membran geblottet. Die PVDF Membran wird anschliessend entnommen und getrocknet. Sie enthält die zweidiemensional getrennten und durch Blot übertragenen Proteine als Spotmuster.

*Beispiel 3:*

**Selektion der Bindungsmoleküle an einem histologischen Schnitt**

Für die Herstellung histologischer Schnitte kann jedes nach dem Stand der Technik und in der Routine histologischer Laboratorien übliche Verfahren benutzt werden. Im Beispiel werden Paraffinschnitte benutzt.

**2.1 Herstellung histologischer Schnitte**

Von dem Paraffinblock, der die Probe enthält, werden an einem Mikrotom 5μm dünne Schnitte in Serie angefertigt, wobei die Serie mindestens 10 Schnitte umfassen sollte. Die Schnitte werden auf dem Wasserbad bei 40°C gestreckt und durch Trocknen auf handelsübliche Glasobjektträger montiert. Um das geschnittene Gewebe von Paraffin zu befreien, wird der Objektträger mit dem Schnitt 2x 10min in jeweils frisches Xylol eingestellt und anschließend durch eine absteigende Alkolreihe (100% Ethanol, 100% Ethanol, 96% Ethanol, 90% Ethanol, 70% Ethanol, 50% Ethanol, Wasser) in Wasser überführt. Die Schnitte werden in Meyer's Hämalaun gefärbt, kurz mit destilliertem Wasser abgewaschen und in Leitungswasser gebläut. Mit Hilfe der Hämalaunfärbung werden die Zellkerne selektiv angefärbt und damit die histologische Struktur unter dem Lichtmikroskop sichtbar.

### 2.2 Blockierung und Inkubation mit der Phagenbibliothek

Die Schnitte werden anschließend 1h bei 37°C mit der Blockierungslösung behandelt (3% Magermilchpulver, Lebensmittelqualität; isotone, phosphatgepufferte Kochsalzlösung (PBS; pH 7,2)). Parallel zu diesem Inkubationsschritt wird die Phagenbibliothek in 500μl Blockierungslösung eingebracht und auf einem leeren Glasobjektträger bei 37° für 1h inkubiert. Nach der Blockierung wird die Blockierungslösung vom Schnitt abgekippt und die Phagenbibliothek von dem leeren Objektträger auf den Objektträger mit dem Schnitt mit einer Pipette transferiert. Die Phagenbibliothek wird 1h bei 37°C mit dem Schnitt inkubiert und anschließend abgekippt. Der inkubierte Objektträger wird nunmehr gewaschen. Dazu wird zunächst 3x5min. bei 37°C mit Blockierungslösung gewaschen, anschließend 3x5min. mit jeweils 50ml PBS bei Raumtemperatur.

### 2.3 Microdissektion an histologischen Schnitten

Im Falle der lasergestützten Microdissektion wird der Objektträger nunmehr nach den Angaben des Herstellers (SL Microtest, Jena) mit einer lasergeeigneten Folie (SL Microtest, Jena) überzogen, die fest mit dem Schnitt verbunden ist. Diese Präparation wird in die Schneidervorrichtung der lasergestützten Microdissektionsapparatur eingespannt. Unter dem Mikroskop werden nun solche Areale, die den Synzytiotrophoblasten der menschlichen Placenta enthalten, mit Hilfe der lasergestützten Microdissektion ausgeschnitten und in Reaktionsgefäße transferiert. An diesen ausgeschnittenen Stücken sind anhängende Phagenantikörper aus der Bibliothek zu finden, bevorzugt solche, die eine spezifische Bindung an den Synzytiotrophoblasten der menschlichen Placenta aufweisen.
Im Falle der mechanischen Microdissektion wird mit geeigneten Glaskapillaren ein kleines Gewebestück aus dem Schnitt herausgelöst und in ein Reaktionsgefäß transferiert

### 2.4 Elution und Amplifikation gebundener Phagen

Im Reaktionsgefäß können diese Phagen nunmehr von dem microdissezierten Material eluiert werden, indem sie 5-10min. mit 0.1M Glycin-Puffer (pH2.2) inkubiert werden. Die eluierten Phagensuspensionen werden anschließend neutralisiert und die neutraliserte Phagensuspension dann benutzt, um neue Populationen von E.coli (z.b. XL1-Blue) zu infizieren.

### 2.5 Reamplifikation eluierter Phagen und zyklische Wiederholung der Selektionsschritte

Die so infizierten Bakterien tragen nunmehr das Merkmal der Ampicillinresistenz und können nach in Lehrbüchern beschriebenen Verfahren (Barbas, Burton, Scott, 2000) nach Superinfektion mit dem Helferphagen M13KO7 in einer Schüttelkultur über Nacht große Mengen von Kopien der eluierten Phagen herstellen.
Diese Phagen können nun aus den Kulturüberständen geerntet und wiederum benutzt werden, um die oben beschriebene Prozedur zur Selektion von Bindungsmolekülen an einem Folgeschnitt zu durchlaufen. Dabei wird anstatt der originären Phagenbibliothek diejenige Phagenpopulation benutzt, die über Nacht aus den eluierten Phagen amplifiziert werden konnte.
Auf diese Weise kann der Prozess der Phagenselektion cyclisch so lange wiederholt werden, bis es zu einer befriedigenden Anreicherung von Phagen gekommen ist, die hochspezifisch an die gewünschte biologische Zielstruktur binden. Mit zunehmender Anzahl durchlaufener Zyklen kann auch die Stringenz durch Verlängerung der einzelnen Waschschritte oder durch Veränderung der Waschlösung selbst erhöht werden, um bevorzug höher affine und besser bindenden Bindungsmoleküle zu erhalten.

### 2.6 Kontrolle des Ergebnisses durch histochemischen Nachweis der Spezifität

Nach der letzten Runde der Isolation von Bindungsmolekülen werden einzelne der isolierten Phagen in E. coli kloniert und diese monoklonalen Phagen einzeln vermehrt und geerntet. Für jeden der so erhaltenen monoklonalen Phagenantikörper kann nun einzeln am Schnitt überprüft werden, ob er die gewünschte Zielstruktur (Synzytiotrophoblast) erkennt.

Dazu wird ein weiterer Serienschnitt pro Phagenklon benötigt. Jeder Schnitt wird einzeln blockiert und mit Phagenantikörper aus je einem Klon inkubiert. Nach der Inkubation wird gewaschen, aber keine Mikrodissektion durchgeführt. Die gebundenen Phagenantikörper werden nunmehr mit einem kommerziellen Antikörper gegen das Hüllprotein VIII filamentöser Phagen (anti-M13, Maus, erhältlich z.B. von AmershamPharmacia Biotech) nachgewiesen. Die gebundenen anti-M13 Antikörper werden nach dem Waschen des Schnittes mit Tris-HCL Puffer (pH 7,6; 3 mal 5 min. bei Raumtermperatur) mit einem polyklonalen Antikörper gegen Maus-Immunglobuline (Schaf anti-Maus, konjugiert mit Merrettich-Peroxidase, z.B. von sigma Finchemikalien, Deisenhofen) 30 min. bei Raumtemperatur inkubiert. Der Schnitt wird danach erneut 3 mal 5 min. gewaschen und der gebundene polyklonale Antikörper durch chromogenen Nachweis der Peroxidase mit Hilfe von Diaminobenzidin und Wasserstoffperoxid als Substrat nachgewiesen. Kommerzielle Kits für dieses Nachweisverfahren sowie geeignete Sekundärantikörper sind z.B. von der Firma sigma, Deisenhofen, erhältlich. Dieses histochemische Verfahren erlaubt die ortsrichtige Visualisierung gebundener Phagenantikörper in einem histologischen Präparat.

## Tabellen

## Tabelle 1: Sequenzen der Primer für die Amplifikation von Vh und Vk sowie die overlap extension PCR

| Acronym | PCR: Vh und Vk |
|---------|----------------|
| | a) Vk |
| CSCVK | 5´GTGGCCCAGGCGGCCCTGACTCAGCCGTCCTCGGTGTC3´ |
| CKJo-B | 5´CGAAGATCTAGAGGACTGACCTAGGACGGTCAGG3´ |
| | b) Vh |
| CSVHo-F | 5´GGTCAGTCCTCTAGATCTTCCGCCGTGACGTTGGACGAG3´ |
| CSCG-B | 5´CTGGCCGGCCTGGCCACTAGTGGAGGAGACGATGACTTCGGTCC3´ |

| | Overlap-extension PCR |
|---------|----------------------|
| CSC-F | 5´GAGGAGGAGGAGGAGGAGGTGGCCCAGGCGGCCGTGACTCAG3´ |
| CSC-B | 5´GAGGAGGAGGAGGAGGAGGAGCTGGCCGGCCTGGCCACTAGTGGAGG3´ |

Tabelle 2:

| Bezeichnung, Antigen und Größe (Anzahl der Transformanten) pro Bibliothek | | |
|---|---|---|
| Phage Display Library (PDL) | Antigen | Transformanten |
| PDL 1-4 | Homogenat reifer menschlicher Placenta | $1.2 \times 10^9$ |
| PDL 5 | Lebende Zellen, Trophoblast-Zellinie AC1-1 | $10^7$ |
| PDL 6 | Lebende Zellen; Trophoblast-Zellinie Jeg-3 | $9.1 \times 10^7$ |
| PDL 7 | Lebende Zellen; Trophoblast-Zellinie AC-1M88 | $8.6 \times 10^7$ |
| PDL 8 | Primärer Zottentrophoblast, frisch isoliert aus einer reifen Placenta | $1.8 \times 10^9$ |
| PDL 9 | Primärer invasiver Trophoblast, frisch isoliert aus Eihäuten einer reifen Placenta | $3.8 \times 10^7$ |
| PDL 10 | Lebende Zellen; Trophoblast-Zellinie AC-1M32 | $3.1 \times 10^7$ |
| PDL 11 | Lebende Zellen; Trophoblast-Zellinie AC-1M59 | $2.1 \times 10^7$ |

Tabelle 2: (fortgesetzt)

| Bezeichnung, Antigen und Größe (Anzahl der Transformanten) pro Bibliothek | | |
|---|---|---|
| **Phage Display Library (PDL)** | **Antigen** | **Transformanten** |
| **PDL 12** | gereinigtes humanes beta-2-Glycoprotein I | $2 \cdot 10^8$ |
| **PDL 15** | Homogenat von Placenta der Ratte | $4.7 \cdot 10^7$ |
| **PDL (kumulative Bibliothek)** | Mischung aller Phagen-Bibliotheken | Gesamt: $4 \cdot 10^9$ |

**EP 1 249 704 A1**

**Patentansprüche**

1. Verfahren zur Selektion von immunologischen Bindungsmolekülen aus einer Bibliothek immunologischer Bindungsmoleküle mit folgenden Schritten:

   - In Kontakt bringen der Bibliothek immunologischer Bindungsmoleküle mit einer zweidimensional angeordneten Probe enthaltend mindestens ein Epitop

- Isolierung der an das mindestens eine Epitop gebundenen immunologischen Bindungsmoleküle.

2. Verfahren nach Anspruch 1, **dadurch gekennzeichnet, dass** die immunologischen Bindungsmoleküle Antikörper oder Antikörperfragmente, insbesondere scFv-Fragmente sind.

3. Verfahren nach Anspruch 1 oder 2, **dadurch gekennzeichnet, dass** die Bibiothek eine Phagen- Bibliothek ist.

4. Verfahren nach einem der Ansprüche 1 bis 3, **dadurch gekennzeichnet, dass** die Bibliothek aus Nicht-Säugetieren, insbesondere Hühnern gewonnen wurde.

5. Verfahren nach einem der Ansprüche 1 bis 4, **dadurch gekennzeichnet, dass** das mindestens eine Epitop durch eine Anordnung in einer zweidimensional angeordneten Probe strukturell verändert wird.

6. Verfahren nach einem der Ansprüche 1 bis 5, **dadurch gekennzeichnet, dass** die zweidimensional angeordnete Probe ein histologischer Schnitt oder ein Blot einer Naturstoff-Elektrophorese ist.

7. Verfahren nach Anspruch 6, **dadurch gekennzeichnet, dass** die Naturstoff-Elektrophorese eine zweidimensionale Elektrophorese ist.

8. Verfahren nach Anspruch 6 oder 7, **dadurch gekennzeichnet, dass** die Naturstoff-Elektrophorese eine Protein-Elektrophorese ist.

9. Verfahren nach Anspruch 6 **dadurch gekennzeichnet, dass** der histologische Schnitt vor dem in Kontakt bringen in eine wässrige Umgebung überführt wird.

10. Verfahren nach einem der Ansprüche 1 bis 9, **dadurch gekennzeichnet, dass** zwei zweidimensional angeordnete Proben nacheinander eingesetzt werden.

11. Verfahren nach Anspruch 10, **dadurch gekennzeichnet, dass** immunologische Bindungsmoleküle durch in Kontakt bringen mit einer ersten zweidimensional angeordneten Probe aus der Bibliothek immunologischer Bindungsmoleküle entfernt werden, um durch in Kontakt bringen mit einer zweiten zweidimensional angeordneten Probe immunologische Bindungsmoleküle für solche Epitope zu selektieren, die die erste zweidimensional angeordnete Probe und die zweite zweidimensional angeordnete Probe unterscheiden.

12. Verfahren nach einem der Ansprüche 1 bis 11, **dadurch gekennzeichnet, dass** Bereiche der zweidimensional angeordneten Probe durch Mikrodissektionseinrichtungen ausgeschnitten werden, um selektierte Bibliotheken von immunologischen Bindungsmolekülen zu erhalten.

13. Verfahren nach einem der Ansprüche 1 bis 12, **dadurch gekennzeichnet, dass** das Selektionsverfahren nach einem der Ansprüche 1 bis 12 zwei oder mehrmals nacheinander durchgeführt wird.

14. Verfahren nach einem der Ansprüche 1 bis 13 , **dadurch gekennzeichnet, dass** die durch das Verfahren nach einem der Ansprüche 1 bis 13 erhaltenen immunologischen Bindungsmoleküle durch Vereinzelung monoklonalisiert werden.

Fig. 1

**Europäisches Patentamt**

# EUROPÄISCHER RECHERCHENBERICHT

Nummer der Anmeldung

EP 01 10 9232

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | VERSMOLD ET AL.: "Antibodies to human placental endothelium can be selected from chicken-derived phage display antibody libraries by panning on paraffin sections." PLACENTA, Bd. 21, Nr. 7, September 2000 (2000-09), Seite A25 XP001042358 * Zusammenfassung * | 1-6,8, 11-14 | G01N33/68 |
| X | WO 99 39210 A (MILLER SAMUEL ;HUMPHERY SMITH IAN (AU)) 5. August 1999 (1999-08-05) * Seite 53, Zeile 24 – Seite 54, Zeile 21; Ansprüche 1,6-8,13,17,29,34-36,41 * | 1-3,6-8, 10,11, 13,14 | |
| E | WO 01 79559 A (BALSAMO JANNE ;LILIEN JACK (US); ELFERINK LISA A (US); KAMHOLZ JOH) 25. Oktober 2001 (2001-10-25) * Anspruch 1 * | 1,3 | |
| A | DE WILDT RUUD M T ET AL: "Antibody arrays for high-throughput screening of antibody-antigen interactions" NATURE BIOTECHNOLOGY, NATURE PUBLISHING, US, Bd. 18, Nr. 9, September 2000 (2000-09), Seiten 989-994, XP002162316 ISSN: 1087-0156 * das ganze Dokument * | 1-14 | **RECHERCHIERTE SACHGEBIETE (Int.Cl.7)** G01N C12N |

-/--

Der vorliegende Recherchenbericht wurde für alle Patentansprüche erstellt

| Recherchenort | Abschlußdatum der Recherche | Prüfer |
|---|---|---|
| DEN HAAG | 10. Januar 2002 | Hoekstra, S |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie,übereinstimmendes Dokument

EPO FORM 1503 03.82 (P04C03)

**Europäisches Patentamt**

**EUROPÄISCHER RECHERCHENBERICHT**

Nummer der Anmeldung

EP 01 10 9232

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | SCHMITZ, U. ET AL.: "Phage display: A molecular tool for the generation of antibodies – A review." PLACENTA, Bd. 21, Nr. Suppl.A, 2000, Seiten S106-S112, XP001042371 * Seite S110, linke Spalte, Absatz 5 – Seite S111, rechte Spalte, Absatz 1 * | 1-14 | |

RECHERCHIERTE SACHGEBIETE (Int.Cl.7)

Der vorliegende Recherchenbericht wurde für alle Patentansprüche erstellt

| Recherchenort | Abschlußdatum der Recherche | Prüfer |
|---|---|---|
| DEN HAAG | 10. Januar 2002 | Hoekstra, S |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie,übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 01 10 9232

In diesem Anhang sind die Mitglieder der Patentfamilien der im obengenannten europäischen Recherchenbericht angeführten
Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-01-2002

| Im Recherchenbericht angeführtes Patentdokument | | Datum der Veröffentlichung | Mitglied(er) der Patentfamilie | | Datum der Veröffentlichung |
|---|---|---|---|---|---|
| WO 9939210 | A | 05-08-1999 | AU | 740830 B2 | 15-11-2001 |
| | | | AU | 2259799 A | 16-08-1999 |
| | | | WO | 9939210 A1 | 05-08-1999 |
| | | | EP | 1051624 A1 | 15-11-2000 |
| WO 0179559 | A | 25-10-2001 | WO | 0179559 A1 | 25-10-2001 |

EPO FORM P0461

Für nähere Einzelheiten zu diesem Anhang : siehe Amtsblatt des Europäischen Patentamts, Nr.12/82